(19) Europäisches Patentamt
European Patent Office
Office européen des brevets

(11) Publication number : **0 559 461 A1**

# (12) EUROPEAN PATENT APPLICATION

(21) Application number : **93301630.5**

(22) Date of filing : **04.03.93**

(51) Int. Cl.[5] : **C08K 5/527,** C08L 23/02, A61L 31/00

(30) Priority : **05.03.92 US 846652**

(43) Date of publication of application : **08.09.93 Bulletin 93/36**

(84) Designated Contracting States : **DE FR GB IT NL**

(71) Applicant : **GENERAL ELECTRIC COMPANY**
**1 River Road**
**Schenectady, NY 12345 (US)**

(72) Inventor : **Lemmen, Timothy Hans**
**Wielewaallaan 22**
**B-2950 Kapellan (BE)**

(74) Representative : **Pratt, Richard Wilson et al**
**London Patent Operation G.E. Technical Services Co. Inc. Essex House 12/13 Essex Street**
**London WC2R 3AA (GB)**

## (54) Enhancing color stability of polyolefin compositions.

(57) A method of enhancing the resistance of polyolefin compositions to sterilizing radiation involves exposing a polyolefin composition comprising a polyolefin resin and a bis(tri-tert-alkylphenoxy)diphophaspiroundecane, including medical articles made therefrom, to sterilizing radiation. The method provides sterilized discoloration resistant medical articles made from the polyolefin compositions.

EP 0 559 461 A1

The present invention relates to enhancing the resistance of polyolefin compositions to sterilizing radiation, and more particularly relates to methods of enhancing the resistance of polyolefin compositions to discoloration upon exposure to sterilizing radiation.

## Description of the Related Art

Polyolefin polymers are well known thermoplastic materials, which due to their properties find use in the medical industry. Polyolefins, however, have a tendency to discolor upon exposure to radiation sterilization procedures, the most prominent of these being gamma radiation.

Accordingly, it is desired to provide a method for enhancing the resistance of polyolefin compositions to discoloration upon exposure to sterilizing radiation.

## Summary of the Invention

The present invention provides a method of enhancing the resistance of polyolefin compositions to sterilizing radiation, the method comprises exposing a polyolefin composition, including medical articles made therefrom, to sterilizing radiation wherein the composition comprises a polyolefin resin and a bis(tri-tert-alkylphenoxy) diphosphaspiroundecane, and preferably a bis(2,4,6-tri-t-alkylphenoxy)diphosphaspiroundecane. The present invention also provides discoloration resistant medical articles made from the polyolefin compositions.

## Detailed Description of the Invention

The polymeric compositions used in the present invention are set out in U.S. Patent 5023285 issued June 11, 1991, inventor William E. Horn, filed July 25, 1988, U.S. Serial No. 07/223,318 which is incorporated herein by reference. The polymeric compositions comprise a polyolefin resin and a bis(tri-tert-alkylphenoxy) diphosphaspiroundecane.

The bis(tri-tert-alkylphenoxy) diphosphaspiroundecane is preferably a 3,9-bis (2,4,6-tri-t-alkylphenoxy)-2,4,8,10-tetraoxa-3 ,9-diphosphaspiroundecane, also known as bis(2,4,6-tri-t-alkylphenyl) pentaerythritol diphosphites, which may be represented by the general formula:

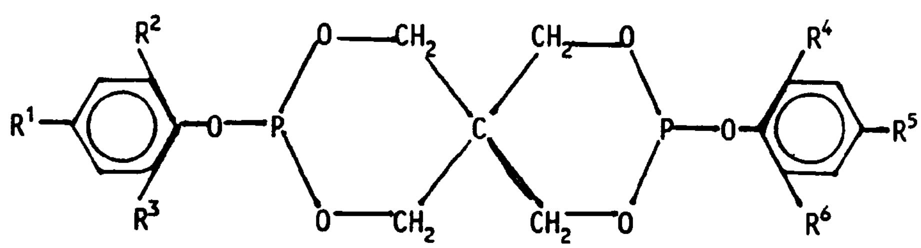

wherein each of $R^1$, $R^2$, $R^3$, $R^4$, $R^5$ and $R^6$ are tertiary ( "tert", "t" ) alkyl moieties.

Examples of such moieties include t-butyl, t-pentyl, 1,1,4,4-tetramethyl butyl, t-octyl, 1-methyl cyclohexyl, t-dodecyl and 2-phenyl-2-propyl.

However, $C_4$ to about $C_{12}$ moieties, such as t-butyl, t-pentyl, t-octyl and t-dodecyl are preferred. Relatively smaller groups, such as t-butyl, t-pentyl, 1-methylcyclohexyl and 1,1,4,4-tetramethyl butyl are more preferred. Tertiary butyl moieties are most preferred. Although any or all of $R^1$,$R^2$,$R^3$,$R^4$,$R^5$ and $R^6$ may be selected to be different, such as in 2,4-di-t-butyl-5-t-pentyl, it is preferred that $R^1$,$R^2$,$R^3$,$R^4$,$R^5$ and $R^6$ be the same.

The diphosphaspiroundecanes of the present invention may be made by means known in the art, such as by the reaction of a tri-t-alkylphenyl with 3,9-dichloro-2,4,8,10-tetroxa-3,9-diphosphaspiro (5.5) undecane (which may be formed by the reaction of pentaerythritol with phosphorous trichloride by means known in the art). For example, 3,9-bis(2,4,6-tri-t-butylphenoxy_-2,4,8,10-tetroxa-3,9-diphosphaspiro (5.5) undecane may be formed by the reaction of 2,4,6-tri-t-butylphenol with 3,9-dichloro-2 ,4,8,10-tetroxa-3,9-diphosphaspiro (5.5) undecane (dichloropentite). Similarly, 3,9-bis-(2,4,6-tri-t-pentyl phenoxy)-2,4,8,10-tetroxa-3,9-diphosphaspiro (5.5) undecane may be formed by the reaction of 2,4,6-tri-t-pentyl phenol with dichloropentite. Other bis(tri-t-alkylphenoxy)-tetroxa-diphosphaspiroundecanes may be formed by reacting the tri-t-alkyphenol corresponding to the desired tri-t-alkylphenoxy group with dichloropentite.

The diphosphaspiroundecanes of the present invention may also be made by reacting a phenol corresponding to the desired tri-t-alkylphenoxy group with phosphorous trichloride to form a tri-t-alkylphenoxy phosphorodichloridite, followed by reaction of the phosphorodichloridite with pentaerythritol to form a bis(tri-t-alkylphenoxy )-tetroxa-diphosphaspiroundecane. For example 2,4,6-tri-t-butylphenol may be reacted with phosphorous trichloride and then with pentaerythritol to form bis(2,4,6-tri-t-butyl -phenoxy)-tetroxa-diphosphaspiroundecane. Similarly, 2,4,6-tri-t-dodecylphenol might be reacted with phosphorous trichloride and then with pentaerythritol to form bis(2,4,6-tri-t-dodecylphenoxy )-tetroxa-diphosphaspiroundecane.

Procedures for forming diphosphaspiroundecanes by the dichloropentite and the phosphorodichloridite routes are known in the art. However, whereas the prior art may show the diphosphaspiroundecane being formed in solution in the presence of an amine, such triethylamine, which serves as an acid acceptor by forming an insoluble hydrochloride salt, it is preferred that the diphosphaspiroundecane be formed in a tri-n-alkylamine, such as tri-n-butylamine, which each n-alkyl group is a $C_3$ or larger n-alkyl moiety, wherein the hydrochloride salt remains in solution and the diphosphaspiroundecane product is substantially insoluble. Preferred processes are explained further in U.S. Patent 5023285 and application U.S. Serial No. 07/223,319, filed 7/25/88, entitled "3,9-Diphosphaspiroundecanes and Process for Making 3,9-Diphosphaspiroundecanes", by S. J. Hobbs, K. J. Sheehan, and W. P. Enlow and published application EP356688 published March 7, 1990, the disclosure of all of which are incorporated herein by reference. Examples of both prior art and preferred processes for making diphosphaspiroundecanes of the invention are shown below in the section entitled "Specific Embodiments".

The present invention also provides a medical article made from the stabilized polymer composition which includes an effective amount of one or more of the bis(tri-t-alkylphenoxy)-tetroxa-diphosphaspiroundecanes described above. An amount of the diphosphaspiroundecanes of the invention is considered to be an "effective amount", when the polymer composition containing the diphosphaspiroundecane of the invention shows improved stability in resistance to discoloration (yellowing) upon exposure to sterilizing radiation in comparison to an analogous polymer composition which does not include a diphosphaspiroundecane of the invention. In most polymer compositions, however, it will be preferred that the diphosphaspiroundecane of the invention be present in an amount equal to about .01 to about 2 parts by weight per 100 parts by weight resin (phr). Amounts of about .01 to about 1 phr are more preferred, although most compositions will contain about 0.025 phr or more. These polymeric compositions are disclosed in U.S. Patent 5023285.

The polyolefin polymers include polymers of monoolefins and diolefins, for example polypropylene, polyisobutylene, polybutene-1, polymethylpentene-1, polyisoprene or polybutadiene, as well as polymers of cycloolefins, for instance of cyclopentene or norbornene, polyethylene (which optionally can be crosslinked), for example high density polyethylene (HDPE), low density polyethylene (LDPE) and linear low density polyethylene (LLDPE) may be used. Mixtures of these polymers, for example mixtures of polypropylene with (PP) polyisobutylene, polypropylene with polyethylene (for example PP/HDPE, PP/LDPE) and mixtures of different types of polyethylene (for example LDPE/HDPE), may also be used. Also useful are copolymers of monoolefins and diolefins with each other or with other vinyl monomers, such as, for example, ethylene/propylene, LLDPE and its mixtures with LDPE, propylene/butene-1, ethylene/hexene, ethylene/ethylpentene, ethylene/heptene, ethylene/octene, propylene/isobutylene, ethylene/butant-1, propylene/butadiene, isobutylene/isoprene, ethylene/alkyl acrylates, ethylene/alkyl methacrylates, ethylene/vinyl acetate (EVA) or ethylene/acrylic acid copolymers (EAA) and their salts (ionomers) and terpolymers of ethylene with propylene and a diene, such as hexadiene, dicyclopentadiene or ethylidene-norbornene; as well as mixtures of such copolymers and their mixtures with polymers mentioned above, for example polypropylene/ethylene-propylene-copolymers, LDPE/EVA, LDPE/EAA, LLDPE/EVA and LLDPE/EAA.

The polymeric compositions may further comprise neutralizers, primary antioxidants, secondary antioxidants and light stabilizers such as hindered amine light stabilizer.

The radiation employed to achieve sterilization of the particular object is ionizing radiation, usually gamma radiation produced from a cobalt-60 or cesium-137 radioactive nuclei. The quantity of gamma radiation required for sterilization is generally 0.5 to about 7 Megarads. A second type of radiation, electron beam radiation, is also suitable for sterilization. Electron beam radiation is produced in a high voltage electron acceleration.

## Examples

Further illustration of this invention is set forth in the following examples. There is no intention to limit the scope of the invention to merely what is shown. Examples 1 - 5 illustrate the present invention. Examples A - Y and AA - AE are comparative examples.

AO1 is 2,4-(bis-di-t-butyl phenyl) pentaerythritol diphosphite sold under the trademark Ultranox 626 by GE Specialty Chemicals, Inc.

AO2 is tri (di-t-butyl phenyl) phosphite sold under the trademark Irgafos 168 by Ciba-Geigy Corporation.

AO3 is represented by the formula:

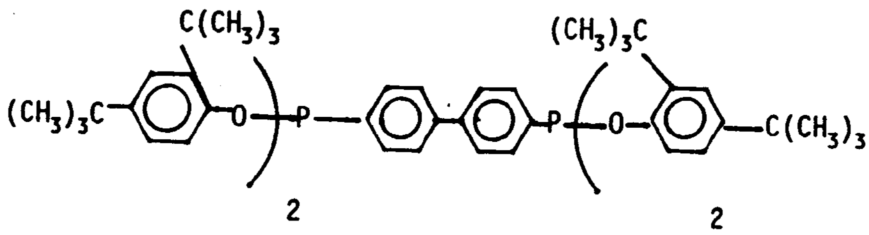

sold under the trademark PEPQ by Adeka Argus.

AO4 is 2,2'-ethylidene bis 4,6 di-t-butyl phenyl fluorophosphate sold under the trademark Ethanox 398 by Ethyl Corporation.

AO5 is trisnonylphenyl phosphite sold under the trademark WESTON TNPP 399 by GE Specialty Chemicals, Inc.

AO7 is bis(2,4,6-tri-t-butylphenoxy)tetraoxa diphosphaspiroundecane having the formula:

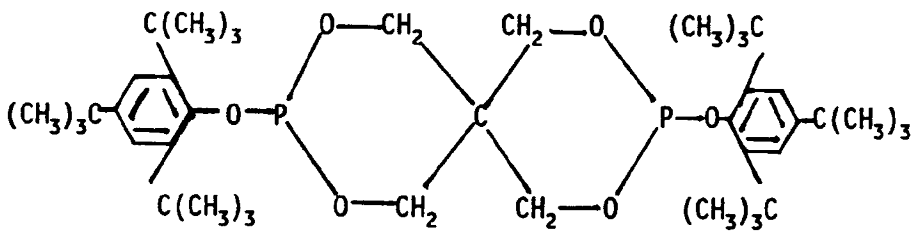

sold under the trademark Ultranox 633 by GE Specialty Chemicals, Inc.

AO6 is distearyl pentaerythritol disphosphite containing approximately 1 percent by weight tri-isopropanol amine sold by GE Specialty Chemicals under the trademark Weston 619®. CaST is calcium stearate. I-1010 is tetrakis (methylene (3,5-di-t-dutyl-4-hydroxy hydrocinnamate) methane sold by Ciba-Geigy under the trademark Irganox 1010®. DSTDP is distearylthiodipropionate. Chimasolb 944LD is poly(16-((1,1,3,3-tetramethylbutyl)amino)-s-triazine-2,4-diyl)((2,2,6,6-tetramethyl-4-piperidyl)imino)hexamethylene((2,2,6,6-t etramethyl -e-piperidyl)imino).
Tinovin 770 is bis(2,2,6,6-tetramethylpiperidyl)sebacate.

All amounts in examples 1 - 29 are parts per million based on the total weight of polypropylene resin. The following examples illustrate the change in yellowing exhibited by various polypropylene compositions having phosphites added thereto.

**TABLE 1**

| | A | B | C | D | E | F | 1 | | |
|---|---|---|---|---|---|---|---|---|---|
| CaST | 1000 | 1000 | 1000 | 1000 | 1000 | 1000 | 1000 | | |
| I-1010 | 1000 | 1000 | 1000 | 1000 | 1000 | 1000 | 1000 | | |
| DSTDP | 2500 | 2500 | 2500 | 2500 | 2500 | 2500 | 2500 | | |
| A01 | -- | 500 | 1000 | -- | -- | -- | -- | | |
| A02 | -- | -- | -- | 1000 | -- | -- | -- | | |
| A03 | -- | -- | -- | -- | 1000 | -- | -- | | |
| A06 | -- | -- | -- | -- | -- | -- | -- | | |
| A04 | -- | -- | -- | -- | -- | 1000 | -- | | |
| A07 | -- | -- | -- | -- | -- | -- | 1000 | | |
| A05 | -- | -- | -- | -- | -- | -- | -- | | |
| | G | H | I | J | K | 2 | | | |
| CaST | 1000 | 1000 | 1000 | 1000 | 1000 | 1000 | | | |
| I-1010 | | | | | | | | | |
| DSTDP | -- | -- | -- | -- | -- | -- | | | |
| A01 | -- | 1000 | -- | -- | -- | -- | | | |
| A02 | -- | -- | 1000 | -- | -- | -- | | | |
| A03 | -- | -- | -- | 1000 | -- | -- | | | |
| A06 | -- | -- | -- | -- | -- | -- | | | |
| A04 | -- | -- | -- | -- | 1000 | -- | | | |
| A07 | -- | -- | -- | -- | -- | 1000 | | | |
| A05 | -- | -- | -- | -- | -- | -- | | | |
| | L | M | N | O | P | Q | 3 | R | S |
| CaST | 1000 | 1000 | 1000 | 1000 | 1000 | 1000 | 1000 | 1000 | 1000 |
| I-1010 | -- | 1000 | 1000 | 1000 | 1000 | 1000 | 1000 | 1000 | 1000 |
| DSTDP | -- | -- | -- | -- | -- | -- | -- | -- | 2500 |
| A01 | -- | -- | 1000 | -- | -- | -- | -- | -- | -- |
| A02 | -- | -- | -- | 1000 | -- | -- | -- | -- | -- |
| A03 | -- | -- | -- | -- | 1000 | -- | -- | -- | -- |
| A06 | -- | -- | -- | -- | -- | 1000 | -- | -- | -- |
| A04 | -- | -- | -- | -- | -- | -- | 1000 | -- | -- |
| A05 | 1000 | -- | -- | -- | -- | -- | -- | 1000 | 1000 |

**TABLE 2**

| | T | U | V | W | X | Y | 4 |
|---|---|---|---|---|---|---|---|
| CaST | 1000 | 1000 | 1000 | 1000 | 1000 | 1000 | 1000 |
| I-1010 | 750 | 750 | 750 | 750 | 750 | 750 | 750 |
| 944LD | -- | 1500 | 1500 | 1500 | 1500 | 1500 | 1500 |
| T770 | -- | -- | -- | -- | -- | -- | -- |
| A01 | -- | -- | 750 | -- | -- | -- | -- |
| A02 | -- | -- | -- | 750 | -- | -- | -- |
| A03 | -- | -- | -- | -- | 750 | -- | -- |
| A06 | -- | -- | -- | -- | -- | -- | -- |
| A04 | -- | -- | -- | -- | -- | 750 | -- |
| A07 | -- | -- | -- | -- | -- | -- | 750 |
| | AA | AB | AC | AD | AE | 5 | |
| CaST | 1000 | 1000 | 1000 | 1000 | 1000 | 1000 | |
| I-1010 | 750 | 750 | 750 | 750 | 750 | 750 | |
| 944LD | -- | -- | -- | -- | -- | -- | |
| T770 | 1500 | 1500 | 1500 | 1500 | 1500 | 1500 | |
| A01 | -- | 750 | -- | -- | -- | -- | |
| A02 | -- | -- | 750 | -- | -- | -- | |
| A03 | -- | -- | -- | 750 | -- | -- | |
| A06 | -- | -- | -- | -- | -- | -- | |
| A04 | -- | -- | -- | -- | 750 | -- | |
| A07 | -- | -- | -- | -- | --- | 750 | |

TABLE 3

| | Yo Before | Yf After | Yf-Yo |
|---|---|---|---|
| A | 7.58 | 21.77 | 14.19 |
| B | 7.17 | 22.71 | 15.54 |
| C | 6.77 | 22.90 | 16.13 |
| D | 7.27 | 24.01 | 16.74 |
| E | 7.17 | 23.83 | 16.66 |
| F | 7.01 | 20.50 | 13.49 |
| 1 | 6.94 | 20.73 | 13.71 |
| G | 6.18 | 16.43 | 10.25 |
| H | 5.97 | 20.54 | 14.57 |
| I | 6.74 | 19.47 | 12.73 |
| J | 6.58 | 19.11 | 12.53 |
| K | 7.33 | 18.66 | 11.33 |
| 2 | 6.49 | 17.47 | 10.98 |
| L | 7.41 | 20.07 | 12.66 |
| M | 6.45 | 20.82 | 14.37 |
| N | 6.32 | 26.94 | 20.62 |
| O | 7.59 | 27.79 | 20.20 |
| P | 6.83 | 27.48 | 20.65 |
| Q | 7.25 | 20.18 | 12.93 |
| 3 | 6.57 | 20.56 | 13.49 |
| R | 7.68 | 19.44 | 10.77 |
| S | 8.20 | 25.05 | 16.85 |
| T | 8.67 | 19.44 | 10.77 |
| U | 8.68 | 20.64 | 11.96 |
| V | 7.75 | 40.27 | 32.52 |
| W | 8.47 | 38.32 | 29.85 |
| X | 7.47 | 40.79 | 33.32 |
| Y | 8.78 | 21.68 | 12.90 |
| 4 | 7.01 | 21.65 | 14.64 |
| AA | 8.13 | 21.14 | 13.01 |
| AB | 6.45 | 38.32 | 31.87 |
| AC | 7.27 | 44.39 | 37.12 |
| AD | 6.46 | 42.33 | 35.87 |
| AE | 7.24 | 22.21 | 14.97 |
| 5 | 6.29 | 21.68 | 13.39 |

Yo is the initial yellowness index of the sample before irradiation. Yf is the final yellowness index of the sample after irradiation. Yf-Yo is the change in yellowness of the sample resulting from irradiation of the sample. Yf and Yo are measurements of yellowness and are measured using an XL-835 Colometer made by Pacific Scientific, Gardner Laboratory Division, Bethesda, Maryland, which was operated in a reflectance mode, spectrum included using a 2· observer.

## Claims

1. A method for inhibiting yellowing of a polyolefin composition upon irradiation sterilization thereof, said method comprising admixing a bis(tri-t-alkylphenoxy)-tetraoxadiphosphaspiroundecane with a polyolefin resin.

2. The method of Claim 1, wherein said bis(tri-t-alkylphenoxy)-tetraoxadiphosphaspiroundecane is bis(2,4,6-tri-t-butylphenoxy)-tetraoxa diphosphaspiroundecane.

3. A method of sterilizing a polyolefin composition wherein said method first comprises admixing a bis(tri-t-alkylphenoxyl)-tetraoxadiphosphaspiroundecane with a polyolefin resin and then further comprises exposing said polyolefin composition to a sterilizing amount of sterilizing radiation.

4. A medical article sufficiently radiated to achieve sterilization, said article comprising a polyolefin polymer and a bis(tri-t-alkylphenoxy)-tetraoxa diphosphaspiroundencane.

5. The article of claim 4 wherein said bis(tri-t-alkylphenoxy)-tetraoxadiphosphaspiroundecane is bis(2,4,6-tri-t-butylphenoxy)-tetraoxa diphosphaspiroundecane.

6. The article of claim 5 wherein said bis ( 2, 4, 6-tri-t-butylphenoxy)-tetraoxadiphosphaspiroundecane is present at a level of from 0.01 to 5.0 weight percent based on the combined total weight of the polyolefin polymer and the bis ( 2, 4, 6-tri-t-butylphenoxy)-tetraoxa diphosphaspiroundecane .

7. A medical article suitable for sterilization by irradiating said article comprising a polyolefin composition comprising polyolefin resin and a bis(tri-t-alkyphenoxy)tetraoxa diphosphaspiroundecane.

8. A medical article as claimed in Claim 1 wherein said bis(tri-t-alkylphenoxy) tetraoxa diphosphaspiroundecane is bis(2,4-6-tri-t-butylphenoxy)-tetraoxa diphosphaspiroundecane.

9. The method of Claim 3 wherein said sterilizing radiation is gamma radiation.

European Patent Office

# EUROPEAN SEARCH REPORT

Application Number

EP 93 30 1630

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,Y | US-A-5 023 285 (WILLIAM E. HORN)<br>& EP-A-0 356 687 (BORG-WARNER SPECIALTY CHEMICALS)<br>* page 12; examples 10-11; tables 1-2 *<br>* claims 1-10 *<br>--- | 1-9 | C08K5/527<br>C08L23/02<br>A61L31/00 |
| Y | EP-A-0 255 097 (ADEKA ARGUS CHEMICAL CO.)<br>* page 40 - page 41; examples 1-2 *<br>* claims 1-2,7-9 *<br>----- | 1-9 | |
| | | | TECHNICAL FIELDS SEARCHED (Int. Cl.5 ) |
| | | | C08K<br>C08L<br>A61L |

The present search report has been drawn up for all claims

| Place of search | Date of completion of the search | Examiner |
|---|---|---|
| THE HAGUE | 07 JUNE 1993 | SIEMENS T. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)